## Europäisches Patentamt

## European Patent Office

## Office européen des brevets

(11) Veröffentlichungsnummer: **0 165 905**
**B1**

# EUROPÄISCHE PATENTSCHRIFT

(45) Veröffentlichungstag der Patentschrift:
08.03.89

(51) Int. Cl.⁴: **C 12 Q 1/04, C 12 Q 1/36**

(21) Anmeldenummer: 85810281.7

(22) Anmeldetag: 18.06.85

(54) **Diagnostisches Testverfahren zum Nachweis von oralen pathogenen Bakteriengemischen.**

(30) Priorität: 18.06.84 CH 2955/84

(43) Veröffentlichungstag der Anmeldung:
27.12.85 Patentblatt 85/52

(45) Bekanntmachung des Hinweises auf die Patenterteilung:
08.03.89 Patentblatt 89/10

(84) Benannte Vertragsstaaten:
CH DE GB LI NL SE

(56) Entgegenhaltungen:
EP-A-0 018 825
EP-A-0 065 137
EP-A-0 097 904
DE-A-2 341 538
GB-A-1 176 968
GB-A-2 031 949

CHEMICAL ABSTRACTS, Band 88, Nr. 23, Juni 1978, Seite 302, Nr. 166658n, Columbus, Ohio, US; P.M. TIERNO et al.: "Differentiation of strains of Staphylococcus epidermidis biotypes 1 and 4 by aminopeptidase profiles", & ANN. CLIN. LAB. SCI. 1978, 8(2), 111-16
CHEMICAL ABSTRACTS, Band 88, Nr. 5, 30. Januar 1978, Seite 230, Nr. 34280y, Columbus, Ohio, US; E.H. PETERSON et al.: "A report on the development of a rapid bacterial identification technique - aminopeptidase profile studies", &

(73) Patentinhaber: The University of Michigan, West Engineering Building 550 East University, Ann Arbor, MI 18109 (US)

(72) Erfinder: Lang, Niklaus P., Dr. med. dent. c/o Zahnmed., Kliniken d. Universität Freiburgstr. 7, CH- 3010 Bern (CH)
Erfinder: Gusberti, Francesco A., Dr. med. dent. c/o Zahn-, med. Kliniken d. Universität Freiburgstr. 7, CH- 3010 Bern (CH)
Erfinder: Syed, Salam A. c/o Zahnmed. Kliniken der, Universität Freiburgstrasse 7, CH- 3010 Bern (CH)
Erfinder: Loesche Walter J, School of Dentistry, Ann Arbor, MI 48109 (US)

(74) Vertreter: Fischer, Franz Josef, c/o Bovard AG Optingenstrasse 16, CH- 3000 Bern 25 (CH)

(56) Entgegenhaltungen: (Fortsetzung)
EDRO SARAP RES. TECH. REP. 1976, 1, PAPER 9-73, 29 PP.

**Beschreibung**

Die vorliegende Erfindung betrifft ein diagnostisches Testverfahren zum Nachweis eines für die oralen Gewebe pathogenen Bakteriengemisches, insbesondere eines Gemisches von mit Gingivitis und Parodontitis vergesellschafteten Bakterien, das für Zahnkaries und Zahnfleischerkrankungen verantwortlich ist.

Das Verfahren beruht auf der enzymatischen Aktivität der in der Plaque enthaltenen Bakterien. Die Zahnkaries und die Zahnfleischerkrankungen dürfen heute als Plaqueinfektionen bezeichnet werden (Loesche 1976). Die Fundstellen der zitierten wissenschaftlichen Publikationen können dem am Schluss der vorliegenden Beschreibung angeführten Literaturverzeichnis entnommen werden.

Im Journal of Clinical Microbiology 14, Seiten 288 - 294 (1981) ist die Bestimmung von oralen Bakterien mittels der von den Bakterien freigesetzten Enzymen beschrieben. Im Gegensatz zum erfindungsgemässen Verfahren müssen hier jedoch die Bakterien zuerst gezüchtet werden und zwar 2 bis 4 Tage unter aeroben Bedingungen.

Die EP-A-18 825 beschreibt ein Verfahren zur Indentifikation von Bakterien aus klinischen Proben wie Urinproben, Rachenabstrichen und -sputums, Wundabstrichen, Stuhl- und Blutproben. Das Verfahren umfasst eine Kombination von verschiedenen Tests, die auf dem an sich bekannten Nachweis von bakteriellen Enzymen mittels zweckmässigen Substraten, wie verschiedenen Aminosäure-β-naphthylamiden, beruht (vgl. Chem. Abstract 88, 1978, Seite 302, 166658). Die GB-A-2 031 949 beschreibt ein ähnliches Verfahren, welches jedoch insbesondere auf den Nachweis von Mikroorganismen der Art der Neisseria abgestimmt ist, wobei das durch die Spaltung des Substrates freigesetzte chromogene Produkt mit einem Diazonium-Nachweisreagens unter Bildung eines Diazofarbstoffes umgesetzt wird.

Es besteht jedoch in keinem der Dokumente des Standes der Technik ein Hinweis, dass die Enzymaktivitätsnachweisverfahren ebenfalls für diagnostische Testverfahren im zahnmedizinischen Bereich geeignet sind. Dies ist nicht selbstverständlich, da die Probemengen bei der Untersuchung von Zahnplaque sehr gering sind.

Bei einer Infektion im klassischen Sinn müssten jedoch Koch's Postulate erfüllt sein:

1.  Ein pathogener Organismus müsste aus der Läsion isoliert und rein gezüchtet werden können.
2.  Dieses Pathogen müsste erfolgreich in einen neuen Wirt eingeführt werden können.
3.  Im neuen Wirt müsste das Pathogen die gleiche Erkrankung hervorrufen.
4.  Der pathogene Mikroorganismus müsste aus dem zweiten Wirt wiederum isoliert werden können.

Während solche Transmissibilitätsuntersuchungen für die Karies beim Hamster erfolgreich waren (Keyes 1960), gelang es bis heute nicht, am Menschen spezifische für die parodontalen Erkrankungen pathogene Bakterien im Sinne einer klassischen Infektion mit Ursache - Wirkung-Beziehung zu verknüpfen. Kulturelle Untersuchungen von Slots (1977a, b, 1978) wie auch longitudinale Untersuchungen dokumentierten, dass es sich bei den Parodontalerkrankungen um opportunistische Erkrankungen handelt. Bei einer solchen können pathogene Bakterien bereits im Zustand der Gesundheit nachgewiesen werden. Durch die Veränderung des Ökosystems auf Grund exogener oder endogener Faktoren werden nun aber für pathogene Keime ideale Wachstumsbedingungen geschaffen, sodass sich diese ungehindert vermehren können und danach prozentual den grössten Anteil der subgingivalen Flora einnehmen. Bei der Therapie von opportunistischen Infektionen wären Pathogene nicht vollständig zu eliminieren, sondern auf eine möglichst kleine Zahl zu reduzieren, sodass sie in ihrer ökologischen Nische einen möglichst geringen und vom Wirt kontrollierbaren Einfluss auf die Gewebe ausüben. Entsprechend wäre die Therapie als erfolgreich zu bezeichnen, wenn es über lange Zeit gelingt, die subgingivale Flora vom Überhandnehmen pathogener Keime zu bewahren.

Wesentliche Voraussetzungen für das Entstehen einer opportunistischen Erkrankung sind:

1.  Das Vorhandensein pathogener Keime
2.  Das Vorhandensein eines für die Vermehrung dieser Keime günstigen Milieus.

Die Assoziationsstudien von Slots (1977a, b, 1978) haben gezeigt, dass im Zustand der gingivalen Gesundheit ebenfalls Gram-negative Anaerobier, so zum Beispiel Bakteroides, (Bacteroides sp.) welche im Tiermodell als Pathogene identifieziert werden konten, vorhanden sind.

Bereits Waerhaug (1955) wies nach, dass durch die kalzifizierten Beläge ein Milieu geschaffen wird, welches die Besiedlung der Bakterien auf dem Zahnstein begünstigt. Vor einigen Jahren hat nun Kornman mit seinen Mitarbeitern (1981) ein Affenmodell entwickelt, mit dem es gelang, solche für die Besiedlung mit pathogenen Keimen begünstigende Faktoren im subgingivalen Ökosystem der parodontalen Tasche zu erzeugen. Durch das Plazieren und wiederholte Nachschieben in apikaler Richtung von Ligaturen mit nachfolgender Plaqueakkumulation konnte eine gingivale Entzündung und als Resultat ein Verlust an parodontalem Attachment induziert werden. Gleichzeitig wurden während verschiedenen Stadien der 4-monatigen Versuchsdauer die subgingivale Bakterienflora qualitativ und quantitativ bestimmt. Es zeigte sich, dass bereits zu Beginn des Experimentes eine, wenn auch geringe Prozentzahl von in schwarzen Kolonien wachsenden Bakteroides (Bacteroides gingivalis) vorhanden war. Diese nahm von 1,3 % wahrend der Entwicklung der Gingivitis auf 6 % zu und erreichte einen Maximalwert von 34,2 % der gesamten züchtbaren Flora gerade zum Zeitpunkt, als klinisch Attachmentverlust und in identisch aufgenommenen Röntgenbildern Knochenverlust

2

EP 0 165 905 B1

dokumentiert werden konnte. Diese Resultate lassen vermuten, dass in der geschützten ökologischen Nische der subgingivalen Plaqueflora durch die sich entwickelnde Gingivitis ein Milieu geschaffen wurde, welches die Proliferation von parodontopathogenen Keime förderte.

In einem ähnlichen klinisch-experimentellen Versuch beim Menschen wurde das Ökosystem durch inkorporierte Füllungsüberschüsse bewusst verändert (Lang, Kiel & Anderhalden 1983). Neun Studenten der Zahnmedizin mit gepflegten Gebissen und klinisch gesunder Gingiva stellten sich für diese Studie freiwillig zur Verfügung. Fünf gegossene MOD-Onlays mit approximal etwa 1 mm überstehenden Rändern wurden 19 bis 27 Wochen lang in Unterkiefermolaren eingesetzt. Darauf wurden diese Onlays in einem Kreuzversuch durch 5 ähnliche Onlays, dieses Mal mit klinisch perfektem Randschluss, ersetzt. Bei den übrigen Probanden wurden zweimal je 5 Onlays in umgekehrter Reihenfolge eingesetzt. Vor der Insertion und in Abständen von 2 bis 3 Wochen danach, wurden subgingivale mikrobiologische Proben entnommen. Eine feine sterile Papierspitze wurde 30 Sekunden lang in den unterhalb der Füllung gelegenen Abschnitt des gingivalen Sulkus eingebracht. Die vorherrschende kultivierbare Flora wurde durch anaerobe Züchtungsmethoden bestimmt. An den Restorationen mit Überschüssen wurde eine subgingivale Flora, die derjenigen einer chronischen Parodontitis glich, gefunden. Erhöhte Prozentanteile Gram-negativer anaerober Bakterien, schwarz-pigmentierter Bakteroides und ein erhöhtes Verhältnis von Anaerobiern zu fakultativen Organismen konnte festgestellt werden. Nach dem Anbringen der Füllungen mit klinisch perfektem Randschluss wurde eine Mikroflora beobachtet, die für das Vorliegen gingivaler Gesundheit oder für eine beginnende Gingivitis charakteristisch war. Schwarz-pigmentierte Bakteroides wurden in nur sehr geringen Proportionen (1,6 - 3,8 %) in diesem Zustand entdeckt. Die Veränderungen in der subgingivalen Mikroflora traten offenbar unabhängig davon auf, ob die Restorationen mit den Überschüssen während der ersten oder während der darauf folgenden gekreuzten Versuchsperiode befestigt worden waren. Klinisch zeigten sich erhöhte Gingival-Indices in den Regionen, in denen sich die Überschüsse befanden. Bluten aus dem gingivalen Sulkus bei vorsichtigem Sondieren trat immer vor dem Erreichen der höchsten Prozente der schwarz-pigmentierten Bakteroides auf. Attachmentverlust wurde vermutlich deshalb nicht beobachtet, weil die Versuchsperiode nicht lange genug angedauert hatte. Zudem wäre ein solcher Versuch vom ethischen Standpunkt aus kaum vertretbar gewesen. Die Veränderungen der subgingivalen Mikroflora nach dem Anbringen der mit Überschüssen versehenen Restorationen dokumentierten einen potentiellen Mechanismus der Einleitung parodontaler Destruktion durch iatrogene Faktoren. Zudem unterstützten die Resultate die opportunistische Natur der parodontalen Plaqueinfektion. Die in schwarzen Kolonien wachsenden Bakteroides, insbesondere Bacteroides gingivalis und Bacteroides intermedius stellen dabei eine Gruppe von Indikatororganismen dar, welche das pathologische Ökosystem der Tasche und damit die Aktivität einer Läsion vermuten lassen.

Diese Gram-negativen Anaerobier dürfen aber nicht als die einzige für die Parodontitis als pathogen in Frage kommenden Mikroorganismen betrachtet werden. Obwohl weitere Querschnittsuntersuchungen die zentrale Rolle dieser Gruppe von Bakterien in der Ätiologie der parodontalen Destruktion dokumentierten (Spiegel et al., 1979, White & Mayrand, 1981), gewannen in der letzten Zeit die bereits von Anthony van Leeuwenhoek (1983) beschriebenen Spirochäten an Bedeutung. Verschiedene klinische Studien (Keyes 1982) zeigten, dass es selbst für den Praktiker möglich ist, ein pathologisches Ökosystem durch die Präsenz hoher Prozentanteile von Spirochäten im Dunkelfeld- oder im Phasenkontrast-Mikroskop zusammen mit einer beachtenswerten Zahl von weissen Blutkörperchen zu identifizieren. Obwohl weder die Spirochäten noch die schwarz-pigmentierten Bakteroidesarten durch Ursache - Wirkung-Beziehungen als klassische Pathogene identifiziert werden können, stellen diese Gruppen von Bakterien äusserst günstige Indikatoren dar, welche das Vorkommen eines pathologischen Geschehens, das heisst eine aktive Gewebsdestruktion vermuten lassen.

Die obigen Ausführungen werden zum besseren Verständnis der Erfindung durch die Figuren näher erläutert. Es zeigt:

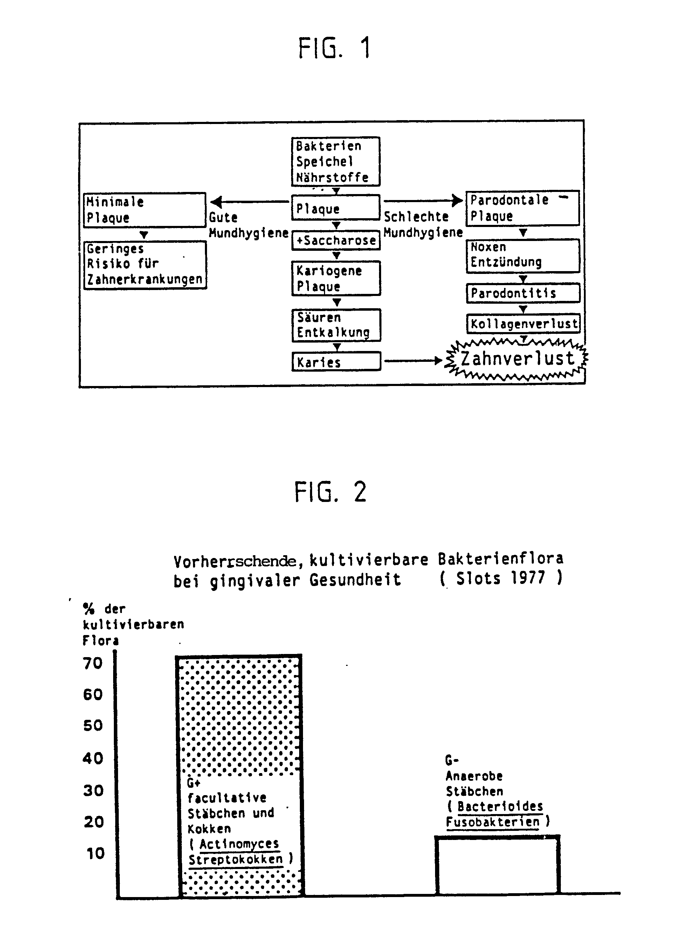

Fig. 1: Verschiedene Plaquetypen und ihre Auswirkungen auf die Mundhöhle.

Fig. 2: Supragingivale Bakterienflora der Plaque vergesellschaftet mit gingivaler Gesundheit (nach Slots 1977a).

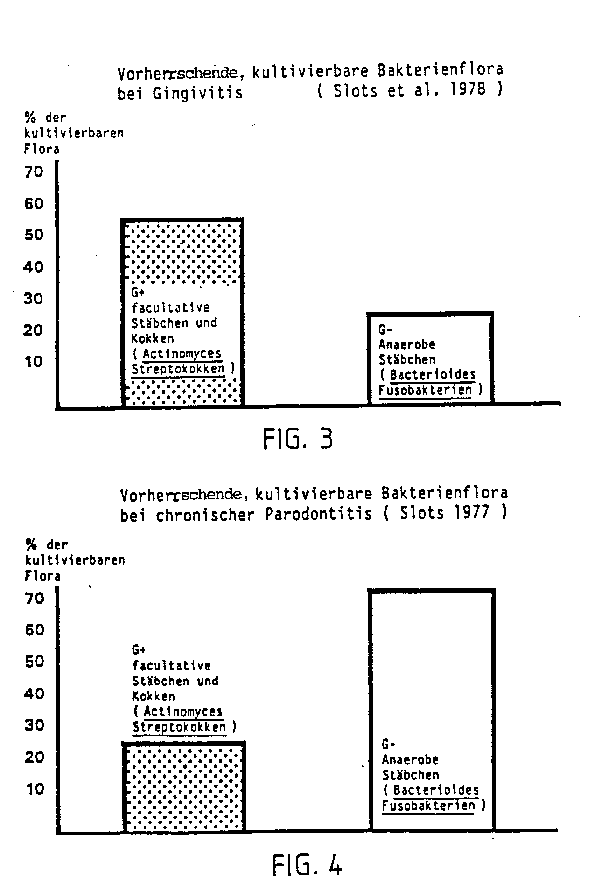

Fig. 3: Bakterienflora der Plaque bei gingivaler Entzündung (nach Slots, Möenbo, Langebaek & Frandsen 1978).

Fig. 4: Subgingivale Bakterienflora der parodontalen Tasche bei Parodontitis (nach Slots 1977b).

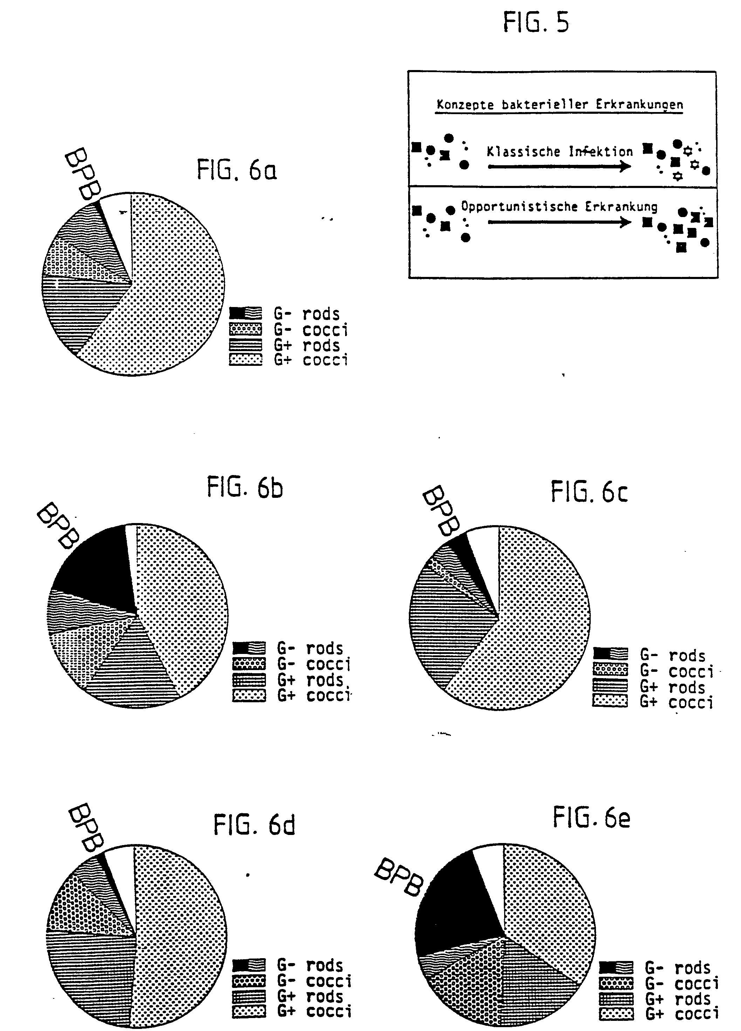

Fig. 5: Modell der a) Klassischen Infektion, bei welcher ein in den Wirt eingedrungenes Pathogen die Erkrankung hervorruft und der b) Opportunistischen Erkrankung, welche durch die Veränderung des Ökosystems zustande kommt. Eine Spezies oder Gruppe von Pathogenen vermehrt sich übermässig und löst die Erkrankung aus.

Fig. 6: Züchtbare, subgingitale Mikroflora: Proportionen der Gram-positiven und Gram-negativen Kokken (Cocci) und Stäbchen (rods). BPB: Schwarz-pigmentierte Bakteroides Kolonien.

Fig. 6a: Vorexperimentelle Werte.

Fig. 6b: Subgingivale Mikroflora nach Inkorporierung von Füllungsüberschüssen. Gram-negative Anaerobier, schwarz-pigmentierte Bakteroides (BPB) signifikant erhöht gegenüber Basisdaten (6a).

Fig. 6c: Subgingivale Mikroflora nach dem Ersetzen der Overlays mit Füllungsüberschüssen mit solchen mit klinisch perfekten Rändern. Gram-negative anaerobe Stäbchen signifikant vermindert gegenüber 6b und den Basisdaten ähnlich (6a).

3

EP 0 165 905 B1

Fig. 6d: Subgingivale Mikroflora bei Overlays mit klinisch perfekten Rändern, vor der Inkorporierung der Füllungsüberschüsse. Keine signifikanten Unterschiede zu den Basisdaten (6a) sowie der ersten Patientengruppe mit perfekten Overlays (6c).

Fig. 6e: Subgingivale Mikroflora nach der Inkorporierung von Füllungsüberschüssen in der Patientengruppe, welche zuerst Overlays mit klinisch perfekten Rändern trug. Gram-negative Anaerobier, schwarzpigmentierte Bakteroides (BPB) signifikant erhöht gegenüber 6d und den Basisdaten (6a).

Die Einleitung jeder Therapie sollte auf möglichst präzisen diagnostischen Angaben basieren. Die in der Parodontologie am meisten angewendeten Methoden sind klinische Messungen der Taschentiefe (Sondierungstiefe) und des Gewebsansatzverlustes, sowie die Erfassung der Plaquemenge und der Entzündung des Zahnfleischrandes (Gingiva) (Glavind & Löe 1967). Diese Messungen dienen zur Beurteilung des pathologischen Zustandes der Parodontalgewebe, basieren jedoch nicht auf den ätiologischen Faktoren, nämlich auf der bakteriellen Plaque allein. In den letzten Jahren wurden verschiedene Versuche unternommen, eine mikrobiologische Differentialdiagnose der Parodontalerkrankungen oder der parodontalen Gesundheit der Patienten zu stellen. Diese schlossen mikroskopische Untersuchungen der bakteriellen Plaque im Dunkelfeld- oder Phasenkontrastmikroskop und mikrobiologische Züchtung der Plaque zur Bestimmung deren Zusammensetzung ein. Die mikroskopische Methode beschränkt sich dabei auf die Analyse der Formen und der Beweglichkeit der Bakterien. Sie verlangt eine genaue Handhabung der Plaqueproben durch erfahrene Leute und ist relativ zeitraubend. Kulturelle Methoden ermöglichen die Bestimmung der qualitativen und quantitativen Zusammensetzung der bakteriellen Plaque. Letztere Methoden sind sehr teuer, aufwendig und zeitraubend und können nur in gut ausgerüsteten Forschungslaboratorien und von speziell geschultem Personal durchgeführt werden.

Zudem ist mit diesen diagnostischen Methoden die Anzahl der zu analisierenden Proben pro Patient beschränkt.

Auf Grund dieser Nachteile und Begrenzungen, es ist schwierig mikrobiologische Methoden mit den erwähnten klinischen diagnostischen Verfahren zu kombinieren.

Die Identifizierung einzelner oraler Bakterien mit enzymatischen Reaktionen wurden in verschiedenen Studien untersucht (Laughon et al. 1982 a, b). Besonders die für die Parodontalerkrankungen als pathogen geltenden Plaquebakterien der Genera Bacteroides, Treponema, Actinomycetes, Capnocytophaga, Fusobacterium und Selenomonas wurden mit chromogenen Substanzen charakterisiert (Syed et al. 1984) (Tabelle 1).

Aufgabe der vorliegenden Erfindung ist es, dem praktizierenden Zahnarzt ein Testsystem zur Verfügung zu stellen, mit welchem er ohne grossen Aufwand aus einer Plaqueprobe ein für die Gewebe pathogenes Bakteriengemisch indentifizieren kann. Das System sollte unter Umgehung der aufwendigen Methoden zur Züchtung von Bakterien mit Zuverlässigkeit ein bakterielles Milieu identifizieren, in welchem bekannte mit Gingivitis und Parodontitis vergesellschaftete Bakterien in hohen Konzentrationen vorkommen. Dadurch kann die Diagnose einer parodontalen Erkrankung mikrobiologisch gestellt und nach Einsetzen der notwendigen Therapie deren Resultat wieder beurteilt werden.

Diese Aufgabe wird durch ein neues diagnostisches Verfahren gelöst, das auf der Darstellung der enzymatischen Aktivität der in der Plaque enthaltenen Bakterien basiert. Es wurde nämlich gefunden, dass durch die Anwendung einer limitierten Zahl von Substraten die Charakterisierung parodontopathischer Plaquebakterien möglich ist.

Gegenstand der vorliegenden Erfindung ist das im Patentanspruch 1 definierte Verfahren.

Mit dem neuen diagnostischen Verfahren werden 4 - 8 ausgewählte chromogene Substrate für die Darstellung enzymatischer Aktivitäten der Plaque verwendet. Der Auswahl dieser Substrate soll die enzymatische Aktivität der von mit Gingivitis und Parodontitis nachgewiesenermassen vergesellschafteten Plaquebakterien wiederspiegeln. Es ist nicht auszuschliessen, dass in Zukunft noch weitere enzymatisch aktive Bakterienspezies und Subspecies aus der parodontalen Plaque isoliert, typisiert und als Pathogen anerkannt werden. Falls diese Mikroorganismen zur Auswahl chromogener weiterer und eventuell relevanterer Substrate führen wurden, könnte das erfindungsgemässe Verfahren ohne weiteres entsprechend ergänzt werden. Die gegenwärtig bevorzugten Substrate sind:

N-α-Benzoyl-D,L-arginin-β-naphthylamid: HCl (Trypsinreaktion) (BANA)
L-Valin-β-naphthylamid (VAL)
L-Leucin-β-naphthylamid·HCL (LEU)
L-Pyrrolidonyl-β-naphthylamid (PYR)
L-Serin-β-naphthylamid (SER)
L-Arginin-β-naphthylamid (ARG)
L-Alanin-β-naphthylamid (ALA)
L-Phenylalanin -β-naphthylamid (PHE)
L-Hydroxy-prolin-β-naphthylamid·HBr
Glycylglycyl-β-naphthylamid·HBr

Die beiden letztgenannten Substrate können auch anstelle von VAL und SER verwendet werden.

Nachstehend werden an Stelle der vollständigen Bezeichnungen der Substrate auch die in Klammern angeführten Abkürzungen verwendet.

Der erfindungsgemässe diagnostische Test basiert auf der Bestimmung der enzymatischen Aktivitäten der Plaquebakterien. Die Gewinnung positiver Reaktionen ist demzufolge von der Anwesenheit bestimmter Bakterien und deren Menge abhängig.

Mit der Zunahme des Schweregrades einer parodontalen Infektion nimmt die Bakterienzahl zu. Zudem werden verhältnissmässig vermehrt pathogene Bakterien nachweisbar. Bakterien, die bei der Gesundheit oder nur leicht entzündetem Zahnfleisch vorhanden sind, kommen aber auch bei schweren Entzündungen vor. Demzufolge sollen mit dem diagnostischen Test, additive Resultate gewonnen werden, indem mit zunehmendem Schweregrad der Entzündung einerseits die Plaquemenge, andererseits aber auch das Vorkommen neuer pathogener Bakterien eine erhöhte Reaktionsintensität und eine vermehrt positive enzymatische Reaktion dokumentiert.

Die Plaqueentnahme am Patienten wird vorzugsweise mit sterilen Parodontalküretten oder mit sterilen Papierspitzen vorgenommen. Ziel dieser Entnahme ist die Gewinnung von an bestimmten Stellen der Zähne oder des Zahnersatzes lokalisierten Plaque in einer grösstmöglichen Menge. Weil pathogene Bakterien am häufigsten an der Front einer Parodontalläsion vorkommen, sollte die Entnahme der Plaque möglichst an der tiefsten Stelle eines Sulcus oder einer Parodontaltasche erfolgen. Es ist vorteilhaft, aber nicht unbedingt notwendig, Speichelkontamination zu vermeiden.

Dies wird mit der Trockenlegung der supragingivalen Entnahmestelle erreicht. Im Gegensatz zu den Plaqueproben für kulturelle Analysen, bei welchen nur subgingivale Plaque entnommen werden muss, ist es mit diesem Verfahren nicht nötig, die supragingivale Plaque vor der Probenentnahme zu entfernen.

Für jeden Patienten können Plaqueproben an verschiedenen Stellen, d. h. in verschiedenen Taschen entnommen werden. Zusätzlich kann auch eine Speichelprobe entnommen werden.

## Beispiel

Die zu analysierende Plaque wird in einem kleinen sterilen, mit einem Deckel versehenen Glasfläschchen gesammelt, das mit 0,5 - 1 ml Hepes-Puffer oder reduzierter Transportflüssigkeit (RTF) (Syed S. & Loesche W.J., 1972, "Appl. Microbiol.", 24, 638 - 644) und mit 5 - 10 gereinigten sterilen Glasperlen (Durchmesser 2 - 3 mm) gefüllt ist.

Die Vorbereitung und Aufbewahrung des obenerwähnten Hepes-Puffers erfolgt folgendermassen:

- Auflösen von 2,38 g N-2-Hydroxyethylpiperazin-N'-2-ethansulfonsäure, (Molekulargewicht 238,3, z. B. "United States Biochemical Corporation", Produkt-Nr. 16926, Katalog 1984) in 1 Liter destilliertem Wasser.
- Einstellen des pH-Wertes mit 0,15M $PO_4$-Puffer auf 7,0.
- Aufbewahren im Kühlschrank bei 4°C.

Bei einer Plaqueentnahme mit sterilen Küretten, wird das Ende dieses Instrumentes mit dem anhaftenden biologischen Material im Glasflaschen geschüttelt, bis die ganze Plaquemenge sich vom Instrument gelöst hat.

Bei einer Plaqueentnahme mit sterilen Papierspitzen, werden dieselben direkt in die im Gläschen enthaltene Flüssigkeit eingetaucht.

## Vorbereitung der Substrate:

Eine prädeterminierte Menge jedes Substrats (meistens 0,016 g) wird zuerst in 1 ml Dimethylsulfoxid in einem Glasröhrchen suspendiert. Zu jedem suspendierten Substrat werden dann 20 ml destilliertes Wasser zugefügt.

Für die Trypsin-ähnliche enzymatische Reaktion, nachstehend als "Trypsintest" bezeichnet, werden 44 mg N-α-Benzoyl-DL-Arginin-β-Naphtylamid HCl (BANA) pro 1 ml Dimethylsulfoxid angewendet. Diese Stock-Lösung soll im Dunkeln aufbewahrt werden (braune Glasflasche oder Glasflasche mit Zinnfolie überzogen). Die fertige Lösung dieses chromogenen Substrats muss gleichen Tags zur Anwendung gelangen, indem zu BANA, Trispuffer pH 7,8 - 8 im Verhältnis von 100 : 1 zugefügt wird.

Die Substratlösungen können bei ca. -20°C und lichtgeschützt aufbewahrt werden oder in Mikrotestplatten (z. B. Minitek$^R$-Platten) in einer Menge von 100 µl pro Vertiefung dispensiert werden. Auf diese Weise vorbereitet Testplatten werden im Kühlschrank bei ca. -20°C und lichtgeschützt bis kurz vor der Anwendung aufbewahrt.

100 µl von jeder zu testenden Substratlösung werden in eine Vertiefung einer sterilen Mikrotestplatte gegeben. Ausser für den Trypsin-Test, dessen Substratlösung gerade vor dem Gebrauch vorbereitet werden muss, können die anderen beschriebenen Substrate vorher in die Platte gefüllt werden, was eine Aufbewahrung bis mindestens 4 Wochen bei -20°C ermöglicht. Wird dieses Vorgehen gewählt, sollen die mit den Substraten vorbereiteten Platten, vor Gebrauch auf Zimmertemperatur gebracht werden.

Jede Plaqueprobe wird in eine Reihe von Vertiefungen zusammen mit den Substraten inkubiert. Dabei wird das kleine Fläschchen mit dem Plaqueinhalt zur Dispersion der Plaque zuerst während 10 Sekunden mit einem Vortexapparat geschüttelt. Danach werden mit einer sterilen Glaspipette ca 100 µl der Plaqueprobe jedem Substrat beigemischt. Die Mikrotestplatte wird zugedeckt und im feuchten Milieu bei 37°C übernacht (mindestens 4 Stunden) inkubiert. Nach Ablauf der Inkubationszeit wird in jede Vertiefung der Mikrotestplatte 100 µl einer chromogenen Reagenzsubstanz (MDDS) zugegeben. In den Vertiefungen für den Trypsin-Test kann die positive Reaktion auch mit der Zugabe von 100 µl 0,1 % Fast Garnet festgesellt werden.

Die Resultate werden innerhalb von fünf Minuten abgelesen. Eine positive Trypsin-Reaktion nach Zugabe von Fast Garnet gibt eine leicht- bis dunkelrote Farbe.

Positive Reaktionen für alle Substrate nach Zugabe von MDDS resultieren in einer roten bis dunkelvioletten Farbe. Negative Resultate sind von gelber bis oranger Farbe.

**Herstellung der MDDS-Flüssigkeit**

| Natriumlaurylsulfat | Sigma | Cat. | Nr. | L-5750 | 1,0 g |
|---|---|---|---|---|---|
| p-Dimethylzimtaldehyd | " | " | " | D-4506 | 0,1 g |
| Ethylen-glycol-monomethylether | " | " | " | E-5378 | 10 ml |
| Triton x 100 | " | " | " | T-6878 | 10 ml |
| Eisessig | | | | | 10 ml |
| Salzsäure (37 %) | | | | | 1 ml |

werden mit destilliertem Wasser versetzt, bis zu einem gesamten Volumen von 1000 ml. Anschliessend wird mit einem Rührwerk ausgiebig gerührt.

Anwendung dieses Tests am Patienten: Mit einer Mikrotestplatte mit 96 Vertiefungen (12 Reihen mit je 8 Vertiefungen) lassen sich folgende Proben herstellen:

Reihe 1 - 10: Plaqueproben aus 10 verschiedenen Parodontaltaschen
11: gesammelte Plaque (Plaque aus verschiedenen Taschen zusammenge "poolt")
12: uninokulierte Kontrolle (in dieser Reihe werden nur Substrate ohne bakterielle Plaque eingefüllt. Nach Inkubation und Zugabe der Reagentien sollte keine positive Farbe entstehen).

**Resultate und deren Bedeutung**

Bis heute wurde nur in wenigen Studien versucht, die enzymatischen bakteriellen Reaktionen oraler Mikroorganismen mit chromogenen Substraten zu untersuchen.

Tabelle 1 gibt die Resultate der 8 enzymatischen Reaktionen bei 135 Bakterienstämmen, die aus Parodontaltaschen am Menschen isoliert wurden. Jeder Bakterienstamm wurde wiederholt analysiert.

Zwei Substrate, nämlich BANA und L-pyrrolidonyl-β-naphthylamid, geben nur bei mit Parodontitis vergesellschafteten Bakterien positive Reaktionen, während andere wie L-Phenylalanin-β-naphthylamid und L-Leucin-β-naphthylamid, die die enzymatischen Reaktionen von vielen oralen Bakterien decken sollten, eher die Zunahme der Plaquemasse in den Parodontaltaschen dokumentieren. Eine weitere Variable, welche bei der Auswahl der Substrate zu berücksichtigen ist, ist die für die Reaktion notwendige bakterielle Masse im Testsystem. Tabelle 2 zeigt bei insgesamt 32 oralen Bakterienstämmen, die ausgerechneten Bakterienzahlen pro ml, die für positive chromogene Reaktionen im Testsystem benötigt werden. Für jede Bakterienspezies und jede chromogene enzymatische Reaktion entspricht die angegebene Zahl dem Durchschnitt der Anzahl der getesteten Stämme einzelner Bakterienspezies. Aus dieser Tabelle ist ersichtlich, dass die für positive enzymatische Reaktionen minimale Bakterienzahl sowohl von den Bakterienspezies als auch von den Substraten abhängen.

Die chromogenen Reaktionen können deshalb von der Anwesenheit bestimmter Bakterienspezies, von der Zahl dieser Bakterien und vom enzymatischen Potential der einzelnen Stämme abhängen. Dabei wurde beim Menschen gezeigt, dass ausgeprägtere enzymatische Reaktionen mit der Zunahme des Schweregrades der Parodontalinfektion einhergehen.

Tabelle 3 bis 9 dokumentieren die Resultate dieser Tests. Bei 24 Probanden wurde eine 3 wöchige Periode ohne mechanische Mundhygiene durchgeführt, während welcher diese aber mit 3 verschiedenen antibakteriellen Lösungen spülten (Tabellen 3 bis 6). 4 weitere Probanden dienten als Kontrolle und erhielten keine Spüllösungen (Tabelle 7).

Bei allen Probanden wurden Plaqueproben an 8 vorher bestimmten Lokalisationen sowohl 14 Tage vor der Studie (-14) und 21 Tage nach Mundhygieneabstinenz (21) entnommen.

Die Resultate des Tages -14 sollen eine Population von jungen Erwachsenen mit durchschnittlich guter Mundhygiene und gesunden Parodontalverhältnissen darstellen. Einige dieser Probanden wiesen jedoch eine Gingivitis auf. Aber auch diese Probanden waren am Anfang der Testperiode klinisch plaquefrei und zeigten gesunde Parodontalverhältnisse, nachdem sie zu Beginn der Studie (Tag 0) eine professionelle Zahnreinigung erhalten hatten. Mit der Mundhygieneabstinenz konnten sich je nach verwendeter desinfizierender Spüllösung unterschiedlich bakterielle Plaques an den Zähnen bilden.

6

Eine Gruppe (C) erhielt eine Spüllösung, welche eine suboptimale antibakterielle Konzentration enthielt und deshalb klinisch mit hohen Plaque- und Gingivitisindices einherging. Die am Tag 21 entnommene Plaque bei dieser Gruppe zeigte auch bedeutend auffallendere chromogene Reaktionen als die anderen zwei Gruppen. Bei den zusätzlichen 4 Probanden, welche ohne Mundhygiene und Spülungen das Experiment bestritten, wurden am Tag 21 auch häufiger chromogene Reaktionen festgestellt (Tabelle 7).

Um die bei Patienten zu erwartenden positiven Reaktionen zu bestimmen, wurden Plaqueproben von 21 sich in der Erhaltungsphase der Parodontalbehandlung befindenden Patienten (Recall-Patienten) und von 7 unbehandelten Parodontitis-Patienten gewonnen (Tabelle 8, 9). Die Durchschnittswerte dieser Untersuchungen zeigten, dass chromogene Reaktionen fast ausschliesslich von bakterieller Plaque aus tiefen Parodontaltaschen (Taschentiefe $\geqslant$ 4mm) herrührten.

Diese Resultate untermauern die Hypothese, dass bei Zunahme des Schweregrades der Parodontalerkrankung (klinisch grössere messbare Taschentiefe) nicht nur mit erhöter Plaquemasse, sondern auch mit vermehrten bestimmbaren enzymatischen bakteriellen Reaktionen einhergeht, welche die Präsenz von pathogenen Bakterien dokumentiert.

So kann der Schweregrad der Parodontalerkrankungen zusätzlich zu den klinisch angewandten Methoden mit chromogenen Reaktionen der bakteriellen Zahnplaque diagnostiziert werden.

**Tabelle 1**

In vitro getestete Bakterienspezies

|  |  | BANA | VAL | PHE | LEU | PYR | SER | ARG | ALA |
|---|---|---|---|---|---|---|---|---|---|
| Bacteroides gingivalis | N = 28 | + | - | - | - | +- | - | +- | - |
| Bacteroides intermedius | N = 24 | - | -+ | - | - | - | - | - | - |
| Bacteroides melaninogenious | N = 17 | - | - | - | - | - | - | -+ | - |
| Actynomyces viscosus | N = 5 | - | - | + | + | - | -+ | - | -+ |
| Actynomyces naeslundii | N = 7 | - | -+ | + | +- | - | -+ | - | -+ |
| Actynomyces odontolyticus | N = 12 | - | -+ | +- | +- | - | +- | -+ | +- |
| Actynomyces israelii | N = 3 | - | - | + | + | - | - | - | + |
| Capnocytophaga sp. | N = 15 | +- | + | + | + | -+ | + | + | + |
| Selenomonas sputigena | N = 3 | - | -+ | - | -+ | - | - | -+ | -+ |
| Veillonella sp. | N = 3 | - | - | - | - | - | - | - | - |
| Fusobacterium sp. | N = 15 | - | - | - | +- | +- | - | -+ | - |

Alle getesteten Bakteriensuspensionen wiesen einen Turbiditätsgrad von Mc Farland 4 - 5 auf.

N = Anzahl der getesteten Bakterienisolate

+   = >95 %

-   = <95 %

+-  = $\geqslant$ 66 % +

-+  = $\geqslant$ 66 % -

**Tabelle 2**

<u>Minimale Bakterienkonzentration für positive enzymatische Reaktionen</u>

| Substrat Bakterium | N | BANA | VAL | PHE | LEU |
|---|---|---|---|---|---|
| Bging | 4 | $1,97 \cdot 10^6$(4)* | - | - | - |
| Bmel | 4 | - | - | - | - |
| Bint | 4 | - | - | - | - |
| AO | 5 | - | $1,4 \cdot 10^{10}$(3) | $1,84 \cdot 10^8$(5) | $1,67 \cdot 10^8$ |
| Anaes | 1 | - | $1,7 \cdot 10^7$ | $4,2 \cdot 10^7$ | $8,1 \cdot 10^6$ |
| Eik | 2 | - | - | - | $10,6 \cdot 10^8$(2) |
| SEL | 3 | - | $3,04 \cdot 10^8$(2) | - | $7,65 \cdot 10^7$(2) |
| Veill | 1 | - | - | - | - |
| FUSO | 5 | - | - | - | $5,13 \cdot 10^7$(5) |
| Capno | 3 | $1,75 \cdot 10^9$(3) | $2,3 \cdot 10^7$(3) | $7 \cdot 10^6$(3) | $1,9 \cdot 10^6$(3) |

**Tabelle 2** Fortsetzung

| Substrat Bakterium | N | PYR | SER | ARG | ALA |
|---|---|---|---|---|---|
| Bging | 4 | $10,88.10^8(3)$ | - | $2,01.10^8(4)$ | - |
| Bmel | 4 | - | - | $5,54.10^8(3)$ | - |
| Bint | 4 | - | - | - | - |
| AO | 5 | - | $1,85.10^7(2)$ | $3,7.10^8(3)$ | $2,32.10^8(5)$ |
| Anaes | 1 | - | - | - | $3,4.10^7$ |
| Eik | 2 | - | - | $9,7.10^7(1)$ | - |
| SEL | 3 | - | - | $1,61.10^8(2)$ | $2,03.10^8(2)$ |
| Veill | 1 | - | - | - | - |
| FUSO | 5 | $1,6.10^8(4)$ | - | $1,48.10^8(4)$ | - |
| Capno | 3 | - | $7,8.10^6(3)$ | $1,95.10^6(3)$ | $7,8.10^5(3)$ |

N = Zahl der getesteten Stämme
*x Bakterienkonzentration für positive Reaktion
( ) Zahl der positiven Bakterienstämme.

| | |
|---|---|
| Bging | = Bacteroides gingivalis |
| Bmel | = Bacteroides melaninogenous |
| Bint | = Bacteroides intermedius |
| AO | = Actynomycetes odontolyticus |
| Anaes | = Actynomycetes naeslundii |
| Eik | = Eikenella corrodens |
| SEL | = Selenomonas sputigena |
| Veill | = Veillomella sp. |
| FUSO | = Fusobacterium sp. |
| Capno | = Capnocytophaga sp. |

**Tabelle 3**

**Experimentelle gingivitis: Tage: 14 Taschen**

Anzahl Patienten:     24
Anzahl Proben:     192

| Enzymatische Reaktion | BANA | VAL | PHE | LEU | PYR | SER | ARG | ALA |
|---|---|---|---|---|---|---|---|---|
| + | 8 | 0 | 58 | 51 | 1 | 1 | 2 | 20 |
| - | 184 | 192 | 134 | 141 | 191 | 191 | 190 | 172 |
| % + | 4,16 | 0,0 | 30,21 | 26,56 | 0,52 | 0,52 | 1,04 | 10,42 |
| % - | 95,83 | 100,0 | 69,79 | 73,44 | 99,48 | 99,48 | 98,96 | 89,58 |

**Experimentielle gingivitis: Tage: 14 Gepoolte Plaque**

Anzahl Patienten:     24
Anzahl Proben:     24

| Enzymatische Reaktion | BANA | VAL | PHE | LEU | PYR | SER | ARG | ALA |
|---|---|---|---|---|---|---|---|---|
| + | 9 | 4 | 21 | 19 | 1 | 3 | 5 | 17 |
| - | 15 | 20 | 3 | 5 | 23 | 21 | 19 | 7 |
| % + | 37,5 | 16,66 | 87,5 | 79,16 | 4,16 | 12,5 | 20,83 | 70,83 |
| % - | 62,5 | 83,33 | 12,5 | 20,83 | 95,83 | 87,5 | 79,16 | 29,16 |

**Tabelle 4**

**Experimentelle gingivitis: Tage: 21 Taschen Gruppe A**

Anzahl Patienten:     8
Anzahl Proben:     64

| Enzymatische Reaktion | BANA | VAL | PHE | LEU | PYR | SER | ARG | ALA |
|---|---|---|---|---|---|---|---|---|
| + | 2 | 3 | 41 | 40 | 0 | 7 | 8 | 32 |
| - | 62 | 61 | 23 | 24 | 64 | 57 | 56 | 32 |
| % + | 3,25 | 4,69 | 64,06 | 62,5 | 0,0 | 10,94 | 12,5 | 50,0 |
| % - | 96,875 | 85,31 | 35,94 | 37,5 | 100,0 | 89,06 | 87,5 | 50,0 |

**Experimentielle gingivitis: Tage: 21 Gepoolte Plaque**

Anzahl Patienten: 8
Anzahl Proben: 8

| Enzymatische Reaktion | BANA | VAL | PHE | LEU | PYR | SER | ARG | ALA |
|---|---|---|---|---|---|---|---|---|
| + | 1 | 3 | 8 | 8 | 1 | 4 | 4 | 8 |
| - | 7 | 5 | 0 | 0 | 7 | 4 | 4 | 0 |
| % + | 12,5 | 37,5 | 100,0 | 100,0 | 12,5 | 50,0 | 50,0 | 100,0 |
| % - | 87,5 | 62,5 | 0,0 | 0,0 | 87,5 | 50,0 | 50,0 | 0,0 |

**Tabelle 5**

**Experimentelle gingivitis: Tage: 21 Taschen Gruppe B**

Anzahl Patienten: 8
Anzahl Proben: 64

| Enzymatische Reaktion | BANA | VAL | PHE | LEU | PYR | SER | ARG | ALA |
|---|---|---|---|---|---|---|---|---|
| + | 0 | 4 | 39 | 35 | 0 | 5 | 11 | 29 |
| - | 64 | 60 | 25 | 29 | 64 | 59 | 53 | 35 |
| % + | 0,0 | 6,25 | 60,94 | 54,69 | 0,0 | 7,81 | 17,19 | 45,31 |
| % - | 100,0 | 93,75 | 39,06 | 45,31 | 100,0 | 92,18 | 82,81 | 54,69 |

**Experimentielle gingivitis: Tage: 21 Gepoolte Plaque Gruppe B**

Anzahl Patienten: 8
Anzahl Proben: 8

| Enzymatische Reaktion | BANA | VAL | PHE | LEU | PYR | SER | ARG | ALA |
|---|---|---|---|---|---|---|---|---|
| + | 2 | 6 | 8 | 8 | 3 | 5 | 6 | 7 |
| - | 0 | 2 | 0 | 0 | 5 | 3 | 2 | 1 |
| % + | 25,0 | 75,0 | 100,0 | 100,0 | 37,5 | 62,5 | 75,0 | 87,5 |
| % - | 75,0 | 25,0 | 0,0 | 0,0 | 62,5 | 37,5 | 25,0 | 12,5 |

**Tabelle 6**

**Experimentelle gingivitis: Tage: 21 Taschen Gruppe C**

Anzahl Patienten: 8
Anzahl Proben: 63

| Enzymatische Reaktion | BANA | VAL | PHE | LEU | PYR | SER | ARG | ALA |
|---|---|---|---|---|---|---|---|---|
| + | 20 | 13 | 56 | 54 | 14 | 16 | 15 | 45 |
| - | 43 | 50 | 7 | 9 | 49 | 47 | 48 | 18 |
| % + | 31,75 | 20,63 | 88,88 | 85,71 | 22,22 | 25,40 | 23,81 | 71,42 |
| % - | 68,25 | 79,37 | 11,11 | 14,28 | 77,77 | 74,60 | 76,19 | 28,57 |

**Experimentielle gingivitis: Tage: 21 Gepoolte Plaque Gruppe C**

Anzahl Patienten:    8
Anzahl Proben:    8

| Enzymatische Reaktion | | BANA | VAL | PHE | LEU | PYR | SER | ARG | ALA |
|---|---|---|---|---|---|---|---|---|---|
| | + | 8 | 7 | 8 | 8 | 5 | 6 | 5 | 8 |
| | - | 0 | 1 | 0 | 0 | 3 | 2 | 3 | 0 |
| % | + | 100,0 | 12,5 | 100,0 | 100,0 | 62,5 | 75,0 | 62,5 | 100,0 |
| % | - | 0,0 | 87,5 | 0,0 | 0,0 | 37,5 | 25,0 | 37,5 | 0,0 |

**Tabelle 7**

**Experimentelle gingivitis: Taschen**

Anzahl Patienten:    4
Anzahl Proben:    32

| Enzymatische Reaktion | | BANA | VAL | PHE | LEU | PYR | SER | ARG | ALA |
|---|---|---|---|---|---|---|---|---|---|
| Tag 21 | + | 3 | 8 | 28 | 25 | 5 | 5 | 9 | 23 |
| | - | 29 | 24 | 4 | 7 | 27 | 27 | 23 | 9 |
| Tag -14 | + | 2 | 4 | 6 | 5 | 0 | 0 | 0 | 2 |
| | - | 30 | 28 | 26 | 27 | 32 | 32 | 32 | 30 |

**Experimentielle gingivitis: Taschen**

Anzahl Patienten:    4
Anzahl Proben:    4

| Enzymatische Reaktion | | BANA | VAL | PHE | LEU | PYR | SER | ARG | ALA |
|---|---|---|---|---|---|---|---|---|---|
| Tag -14 | + | 1 | 0 | 2 | 2 | 0 | 0 | 0 | 0 |
| | - | 3 | 4 | 2 | 2 | 4 | 4 | 4 | 4 |
| Tag 21 | + | 3 | 4 | 4 | 4 | 3 | 3 | 4 | 4 |
| | - | 1 | 0 | 0 | 0 | 1 | 1 | 0 | 0 |

**Tabelle 8**

**Recall - Patienten: Taschentiefe ⩽ 4 mm**

Anzahl Patienten:    21
Anzahl Proben:    142

| Enzymatische Reaktion | | BANA | VAL | PHE | LEU | PYR | SER | ARG | ALA |
|---|---|---|---|---|---|---|---|---|---|
| | + | 16 | 3 | 41 | 35 | 0 | 2 | 3 | 20 |
| | - | 126 | 139 | 101 | 107 | 142 | 140 | 139 | 122 |
| % | + | 11,27 | 2,11 | 28,87 | 24,65 | 0,0 | 1,41 | 2,11 | 14,08 |
| % | - | 88,73 | 97,89 | 71,13 | 75,35 | 100,0 | 98,59 | 97,89 | 85,92 |

**Recall - Patienten: Taschentiefe ⩽ 5 mm**

Anzahl Patienten:    21
Anzahl Proben:    24

| Enzymatische Reaktion | | BANA | VAL | PHE | LEU | PYR | SER | ARG | ALA |
|---|---|---|---|---|---|---|---|---|---|
| | + | 7 | 0 | 9 | 10 | 2 | 0 | 2 | 8 |
| | - | 17 | 24 | 15 | 14 | 22 | 24 | 22 | 16 |
| % | + | 29,16 | 0,0 | 37,5 | 41,66 | 8,33 | 0,0 | 8,33 | 33,33 |
| % | - | 70,83 | 100,0 | 62,5 | 58,33 | 91,66 | 100,0 | 91,66 | 66,66 |

**Recall - Patienten: Geboolte Plaque**

Anzahl Patienten:     21
Anzahl Proben:       21

| Enzymatische Reaktion | | BANA | VAL | PHE | LEU | PYR | SER | ARG | ALA |
|---|---|---|---|---|---|---|---|---|---|
| | + | 8 | 1 | 20 | 18 | 1 | 1 | 1 | 12 |
| | - | 13 | 20 | 1 | 3 | 20 | 20 | 20 | 9 |
| % | + | 38,10 | 4,76 | 95,24 | 85,71 | 4,76 | 4,76 | 4,76 | 57,14 |
| % | - | 61,90 | 95,24 | 4,76 | 14,29 | 95,24 | 95,24 | 95,24 | 42,86 |

**Tabelle 9**

**Unbehandelte Periodontitis - Taschentiefe ≤ 4 mm**

Anzahl Patienten:     7
Anzahl Proben:       15

| Enzymatische Reaktion | | BANA | VAL | PHE | LEU | PYR | SER | ARG | ALA |
|---|---|---|---|---|---|---|---|---|---|
| | + | 8 | 1 | 13 | 12 | 0 | 0 | 0 | 8 |
| | - | 7 | 14 | 2 | 3 | 15 | 15 | 15 | 7 |
| % | + | 53,33 | 6,66 | 86,66 | 80,0 | 0,0 | 0,0 | 0,00 | 53,33 |
| % | - | 46,60 | 93,33 | 13,33 | 20,0 | 100,0 | 100,0 | 100,0 | 46,66 |

**Unbehandelte Periodontitis - Taschentiefe ≥ 5 mm**

Anzahl Patienten:     7
Anzahl Proben:       41

| Enzymatische Reaktion | | BANA | VAL | PHE | LEU | PYR | SER | ARG | ALA |
|---|---|---|---|---|---|---|---|---|---|
| | + | 35 | 5 | 36 | 37 | 4 | 2 | 10 | 34 |
| | - | 6 | 36 | 5 | 4 | 37 | 39 | 31 | 7 |
| % | + | 85,37 | 12,20 | 87,80 | 90,24 | 9,76 | 4,88 | 24,39 | 82,92 |
| % | - | 14,63 | 87,80 | 12,20 | 9,76 | 90,24 | 95,12 | 75,61 | 17,07 |

**Unbehandelte Periodontitis - gepoolte Plaque**

Anzahl Patienten:     7
Anzahl Proben:       7

| Enzymatische Reaktion | | BANA | VAL | PHE | LEU | PYR | SER | ARG | ALA |
|---|---|---|---|---|---|---|---|---|---|
| | + | 7 | 3 | 6 | 7 | 2 | 3 | 4 | 7 |
| | - | 0 | 4 | 1 | 0 | 5 | 4 | 3 | 0 |
| % | + | 100,0 | 42,86 | 85,71 | 100,0 | 28,57 | 42,86 | 57,14 | 100,0 |
| % | - | 0,0 | 57,14 | 14,29 | 0,0 | 71,43 | 57,14 | 42,86 | 0,0 |

**Literaturverzeichnis**

1. Glavind, L. & Löe, H., 1967: Errors in the clinical assessment of periodontal destruction.
J. Periodont. Res. 2:179-184
2. Keyes, P.H., 1960: The infectious and transmissible nature of experimental dental caries.
Archs. Oral Biol. 1:304-320
3. Keyes, P.H., 1982: Microbiologically modulated periodontal therapeutics: an introduction.
Quintessence Int. 13(12):1321-1325
4. Kornman, K.S., Holt, S.C. & Robertson, P.B., 1981: The microbiology of ligature-induced periodontitis in the cynomolgus monkey.
J. Periodont.Res. 16:363-371
5. Lang, N.P., Kiel, R.A. & Anderhalden, K., 1983: Clinical and microbiological effects of subgingival restorations with overhanging or clinically perfect margins.
J.Clin.Periodontol. 10:563-578
6. Laughon, B.E., Syed, S.A. & Loesche, W.J., 1982: System for identification of Bacteroides spp, Capnocytophaga spp. and Spirochetes of oral origin.
J. Clin. Microbiol. 15:97
7. Laughon, B.E., Syed, S.A. & Loesche, W.J, 1982: Rapid identification of Bacteroides gingivalis.
J.Clin.Microbiol. 15:345.
8. Löe, H. & Silness, J., 1963: Periodontal disease in pregnancy. J. Prevalence and Severity.
Acta odont.Scand. 21:533-551
9. Loesche W.J., 1976. Chemotherapy of dental plaque infections.
Oral Sciences Rev. Melcher, A.H. & Zarb, G.A. (eds), Munksgaard Copenhagen 9:65-107
10. Silness, J. & Löe, H., 1964: Periodontal disease in pregnancy. II. Correlation between oral hygiene and periodontal condition.
Acta odont.Scand. 22:121-135
11. Slots, J., 1977a: Microflora in the healthy gingival sulcus in man.
Scand.J.Dent.Res. 85:247-254
12. Slots, J., 1977b: The predominant cultivable flora of advanced periodontitis.
Scand.J.Dent.Res. 85:114-121
13. Slots, J., Möenbo, D., Langebaek, J. & Frandsen, A., 1978: Microbiota of gingivitis in man.
Scand.J.Dent.Res. 86:174-181
14. Spiegel, C.A., Hayduk, S.E., Minah, G.E. & Krywolap, G.N., 1979: Black-pigmented Bacteroides from clinically characterized periodontal sites.
J.Periodont.Res. 14:376-382
15. Syed, S.A., Loesche, W.J., Gusberti, F.A. & Lang, N.P., 1984: Rapid characterization of periodontopathic bacteria by chromogene substrats.
J.Dent.Res. 63:Special Issue/Abstracts 263. Abstract No. 834.
16. Tal, M., 1981: Periodontal disease and oral hygiene described by Antonio van Leeuwenhoeck.
J.Periodontol. 51:668-669
17. Waerhaug, J., 1955: Microscopic demonstration of tissue reaction incident to removal of subgingival calculus.
J.Periodontol. 26:26-29
18. White, D. & Mayrand, D., 1981: Association of oral Bacteroides with gingivitis and adult periodontitis.
J.Periodont.Res. 16:259-265

**Patentansprüche**

1. Diagnostisches Verfahren zum Nachweis eines Gemisches von mit Gingivitis und Parodontitis vergesellschafteten pathogenen, oralen Plaque-Bakterien, dadurch gekennzeichnet, dass eine orale Plaqueprobe in einem sterilen, verschliessbaren Glasfläschchen gesammelt wird, das 0,5 - 1 ml Hepes-Puffer oder reduzierte Transportflüssigkeit und 5 - 10 gereinigte, sterile Glasperlen mit einem Durchmesser von 2 - 3 mm enthält, und dass das Fläschchen etwa 10 Sek. intensiv geschüttelt wird, der Inhalt in 4 - 8 Teile zu 100 µl aufgeteilt und jeder Teil auf enzymatische Aktivität getestet wird, indem jeder Probeteil mit einem chromogenen Substrat gemischt und inkubiert wird, wobei die Substrate aus der Gruppe der folgenden Substrate ausgewählt sind:

N-α-Benzoyl-D,L-arginin-β-naphthylamid·HCl;
L-Valin-β-naphthylamid;
L-Leucin-β-naphthylamid·HCl;
L-Pyrrolidonyl-β-naphthylamid;
L-Serin-β-naphthylamid;
L-Arginin-β-naphthylamid;

L-Alanin-β-naphthylamid;
L-Phenylalanin-β-naphthylamid;
L-Hydroxy-prolin-β-naphthylamid und
Glycylglycyl-β-naphthylamid,

welche Substrate von Enzymen sind, die von im nachzuweisenden Gemisch enthaltenen pathogenen Bakterien freigesetzt werden, wobei die positive enzymatische Reaktion mittels angegriffener Substrate nachgewiesen wird, die durch Zugabe chromogener Reagenzsubstanzen veränderte Lichtabsorptionswerte aufweisen und die mehrheitlich positive enzymatische Reaktion das Vorhandensein eines pathogenen Bakteriengemisches anzeigt.

2. Verfahren nach Anspruch 1, dadurch gekennzeichnet, dass die Substrate als Lösungen verwendet werden, die erhältlich sind durch Suspension von etwa 0,016 g/ml Substrat in Dimethylsulfoxid und anschliessendem 20-fachem Verdünnen dieser Lösung mit destilliertem Wasser.

3. Verfahren nach Anspruch 1, dadurch gekennzeichnet, dass eine Lösung von N-α-Benzoyl-D,L-arginin-β-naphthylamid verwendet wird, die erhältlich ist durch Suspension von 44 mg/ml des Substrates in Dimethylsulfoxid und anschliessendem 100-fachem Verdünnen dieser Lösung mit Trispuffer mit einem pH-Wert von 7,8 - 8,0, wobei diese Lösung innerhalb eines Tages zu verwenden ist.

4. Verfahren nach Anspruch 2 oder 3, dadurch gekennzeichnet, dass die Substratlösungen in Mikrotestplatten in Mengen von 100 µl pro Vertiefung dispensiert werden.

5. Ausrüstung für die Durchführung des Testverfahrens nach einem der Ansprüche 1 - 4, dadurch gekennzeichnet, dass sie folgende Bestandteile in lagerfähig verpackter Form aufweist: 4 - 8 Substrate aus der Gruppe:

N-α-Benzoyl-D,L-arginin-β-naphthylamid·HCl;
L-Valin-β-naphthylamid;
L-Leucin-β-naphthylamid·HCl;
L-Pyrrolidonyl-β-naphthylamid;
L-Serin-β-naphthylamid;
L-Arginin-β-naphthylamid;
L-Alanin-β-naphthylamid;
L-Phenylalanin-β-naphthylamid;
L-Hydroxy-prolin-β-naphthylamid und
Glycylglycyl-β-naphthylamid

für die Bestimmung der enzymatischen Aktivität in einer oralen Plaqueprobe, entweder als lagerstabile verbrauchsfertige Lösungen mit vorbestimmten Konzentrationen oder als mengenmässig abgemessene Komponenten für die Zubereitung der Lösungen unmittelbar vor dem Verbrauch und Behältnisse zum Sammeln und Vorbereiten der Plaqueproben.

6. Ausrüstung nach Anspruch 5, dadurch gekennzeichnet, dass sie als Behältnis eine Mikrotestplatte umfasst, die Vertiefungen aufweist, wobei die Substratpräparate mindestens teilweise in den Vertiefungen vorliegen.

## Claims

1. Diagnostic procedure for detecting a mixture of pathogenic oral plaque bacteria associated with gingivitis and parodontitis, characterized in that an oral plaque specimen is collected in a sterile, small glass bottle containing 0.5 - 1 ml of HEPES buffer or reduced transport liquid and from 5 - 10 cleaned, sterile glass beads having a diameter of from 2 - 3 mm, and that the small bottle is thoroughly shaken for about 10 sec., the contents are divided up into from 4 - 8 parts of 100 µl each, and each part is tested for enzymatic activity by mixing each part specimen with a chromogenic substrate and incubating it, the substrates being chosen from the group of the following substrates:

N-α-benzoyl-D,L-arginine-β-naphthylamide·HCl;
L-valine-β-naphthylamide;
L-leucine-β-naphthylamide·HCl;
L-pyrrolidonyl-β-naphthylamide;
L-serine-β-naphthylamide;
L-arginine-β-naphthylamide;
L-alanine-β-naphthylamide;
L-phenylalanine-β-naphthylamide;
L-hydroxy-proline-β-naphthylamide and
glycylglycyl-β-naphthylamide,

which are substrates of enzymes that are released by pathogenic bacteria contained in the mixture to be detected, the positive enzymatic reaction being detected by means of attacked substrates which, through the addition of chromogenic reagents, have modified light-absorption values, and the substantially positive enzymatic reaction indicates the presence of a pathogenic bacterial mixture.

2. Procedure according to claim 1, characterized in that the substrates are used as solutions obtainable through suspension of about 0.016 g/ml of substrate in dimethylsulfoxide and subsequent 20-fold dilution of this solution with distilled water.

3. Procedure according to claim 1, characterized in that a solution of N-α-benzoyl-D,L-arginine-β-naphthylamide is used, which is obtainable through suspension of 44 mg/ml of the substrate in dimethylsulfoxide and subsequent 100-fold dilution of this solution with TRIS buffer having a pH value of from 7.8 - 8.0, this solution to be used within one day.

4. Procedure according to claim 2 or 3, characterized in that the substrate solutions are dispensed in microtest plates in amounts of 100 µl per well.

5. Kit for carrying out the test procedure according to one of the claims 1 - 4, characterized in that that it has the following components in storably packed form: 4 - 8 substrates from the group:

N-α-benzoyl-D,L-arginin-β-naphthylamide·HCl;
L-valine-β-naphthylamide;
L-leucine-β-naphthylamide·HCl;
L-pyrrolidonyl -β-naphthylamide;
L-serine-β-naphthylamide;
L-arginine-β-naphthylamide;
L-alanine-β-naphthylamide;
L-phenylalanine-β-naphthylamide;
L-hydroxy-proline-β-naphthylamide and
glycylglycyl-β-naphthylamide,

for determination of the enzymatic activity in an oral plaque specimen, either as shelf-stable, ready-to-use solutions of predetermined concentrations or as quantitatively measured-out components for the preparation of the solutions immediately prior to use, and containers for collecting and preparing the plaque specimens.

6. Kit according to claim 5, characterized in that as a container it comprises a microtest plate having wells, the substrate preparations being present at least partially in the wells.

## Revendications

1. Méthode de diagnostic pour la détection d'un mélange de plaques bactérielles buccales et pathogènes associées à la gingivite et à la parodontite, caractérisée en ce qu'un échantillon de plaque orale est recueilli dans une fiole de verre stérile pouvant être bouchée et contenant 0,5 - 1 ml de solution tampon Hepes ou de liquide de transport réducteur et 5 - 10 perles de verre nettoyées et stériles d'un diamètre de 2 - 3 mm et en ce que la fiole est secouée intensivement pendant environ 10 secondes, répartie en 4 - 8 parties à 100 µl et chaque partie testée quant à son activité enzymatique, chaque partie d'échantillon étant mélangée et incubée avec un substrat chromogène, tiré du groupe de substrats suivant:

N-α-Benzoyle-D, L-arginine-α-naphtylamide·HCl;
L-Valine-β-naphthylamide;
L-Leucine-β-naphthylamide·HCl;
L-Pyrrolidonyle-β-naphthylamide;
L-Serine-β-naphthylamide;
L-Arginine-β-naphthylamide;
L-Alanine-β-naphthylamide;
L-Phenylalanine-β-naphthylamide;
L-Hydroxy-proline-β-naphthylamide und
Glycylglycyle-β-naphthylamide,

lesquels substrats viennent d'enzymes qui sont libérés de bactéries pathogènes contenues dans le mélange à détecter, la réaction enzymatique positive étant détectée au moyen de substrats attaqués qui présentent des valeurs d'absorption de la lumière différente par rajout de substances de réactifs chromogènes et la réaction enzymatique positive à plusieurs reprises montrant la présence d'un mélange bactérien pathogène.

2. Methode selon la revendication 1, caractérisée en ce que les substrats sont utilisés en tant que solutions qui sont obtenues par suspension d'environ 0,016 g/ml de substrat dans le diméthylsulfoxide, puis par dilution de cette solution dans 20 volumes d'eau distillée.

3. Méthode selon la revendication 1, caractérisée en ce qu'une solution de N-α-benzoyle-D, L-arginine-β-naphthylamide est utilisée, laquelle est obtenue par suspension de 44 mg/ml de substrat dans le diméthylsulfoxide, puis par dilution de cette solution dans son volume de solution tampon "Tris" ayant un pH de 7,8 - 8,0, cette solution résultante devant être utilisée dans la période d'une journée.

4. Méthode selon la revendication 2 ou 3, caractérisée en ce que les solutions de substrats sont dispensibles dans des micro-plaquettes-échantillon par quantités de 100 ml par cavité.

5. Equipement pour le déroulement de la méthode d'essai selon l'une des revendications 1 à 4, caractérisé en ce qu'il présente les constituants suivants dans un emballage pouvant être stocké: 4 - 8 substrats du groupe:

N-α-Benzoyle-D, L-arginine-β-naphthylamide·HCl;
L-Valine-β-naphthylamide;
L-Leucine-β-naphthylamide·HCl;
L-Pyrrolidonyle-β-naphthylamide;
L-Serine-β-naphthylamide;
L-Arginine-β-naphthylamide;
L-Alanine-β-naphthylamide;
L-Phenylalanine-β-naphthylamide;
L-Hydroxy-proline-β-naphthylamide et
Glycylglycyle-β-naphthylamide

pour la détermination de l'activité enzymatique dans une plaque d'échantillon orale, soit en tant que solution ayant une concentration prédéterminée pouvant être stockée et prête à l'utilisation, soit en tant que composant en quantité mesurée pour la préparation des solutions immédiatement avant l'utilisation et de contenants pour recueillir et préparer les échantillons de plaques.

6. Equipement selon la revendication 5, caractérisé en ce qu'il comprend en tant que contenant une micro-plaquette-test qui presente des cavités, les préparations de substrats se trouvant au moins en partie dans les cavités.

## FIG. 1

Bakterien / Speichel / Nährstoffe

Gute Mundhygiene ← Plaque → Schlechte Mundhygiene

Minimale Plaque

Geringes Risiko für Zahnerkrankungen

+Saccharose

Kariogene Plaque

Säuren Entkalkung

Karies → Zahnverlust

Parodontale Plaque

Noxen Entzündung

Parodontitis

Kollagenverlust

## FIG. 2

Vorherrschende, kultivierbare Bakterienflora bei gingivaler Gesundheit ( Slots 1977 )

% der kultivierbaren Flora

70
60
50
40
30
20
10

G+ facultative Stäbchen und Kokken ( Actinomyces Streptokokken )

G- Anaerobe Stäbchen ( Bacteroides Fusobakterien )

Vorherrschende, kultivierbare Bakterienflora
bei Gingivitis ( Slots et al. 1978 )

FIG. 3

Vorherrschende, kultivierbare Bakterienflora
bei chronischer Parodontitis ( Slots 1977 )

FIG. 4

3

## FIG. 5

Konzepte bakterieller Erkrankungen

Klassische Infektion

Opportunistische Erkrankung

## FIG. 6a

BPB

G- rods
G- cocci
G+ rods
G+ cocci

## FIG. 6b

BPB

G- rods
G- cocci
G+ rods
G+ cocci

## FIG. 6c

BPB

G- rods
G- cocci
G+ rods
G+ cocci

## FIG. 6d

BPB

G- rods
G- cocci
G+ rods
G+ cocci

## FIG. 6e

BPB

G- rods
G- cocci
G+ rods
G+ cocci